# EUROPEAN PATENT SPECIFICATION

(11) **EP 4 304 529 B1**
(45) Date of publication and mention of the grant of the patent: **25.03.2026**
(21) Application number: 22713142.2
(22) Date of filing: 07.03.2022
(51) Int. Cl.: A61F 2/24

(54) **ANNULOPLASTY RING AND TETHER ADJUSTMENT SYSTEM**
ANNULOPLASTIERING UND GURTEINSTELLUNGSSYSTEM
ANNEAU D'ANNULOPLASTIE ET SYSTÈME DE RÉGLAGE D'ATTACHE

(30) Priority: 09.03.2021 US 202163158821 P
(43) Date of publication of application: 17.01.2024
(73) Proprietor: Edwards Lifesciences Corporation, Irvine, CA 92614-5688 (US)
(72) Inventor: CONKLIN, Brian S., Orange, CA 92867 (US)
(74) Representative: Eisenführ Speiser
(86) International application number: PCT/US2022/019176
(87) International publication number: WO 2022/192151

(56) References cited:
- WO-A2-2007/100408
- US-A1- 2003 083 742

## Description

### TECHNICAL FIELD

The present invention relates to an annuloplasty ring and tether adjustment system and a simulation method of deployment and use.

### BACKGROUND

In vertebrate animals, the heart is a hollow muscular organ having four pumping chambers: the left and right atria and the left and right ventricles, each provided with its own one-way valve. The natural heart valves are identified as the aortic, mitral (or bicuspid), tricuspid and pulmonary, and each has flexible leaflets that coapt against each other to prevent reverse flow.

With functional mitral regurgitation caused by ischemia or dilated cardiomyopathy, the mitral valve itself is normal, but regurgitation through a gap in the mitral valve occurs as a result of abnormal tethering forces caused by displacement of the papillary muscles due to enlargement of the left ventricle. Published estimates show that ischemic mitral regurgitation (IMR) occurs in 20-30% following a myocardial infarction. It has also been shown that severe mitral regurgitation is associated with a 3-fold increase in heart failure and a 1.6-fold increase in mortality at 5 years.

Various surgical techniques may be used to repair a diseased or damaged valve or heart muscle. A commonly used repair technique effective in treating incompetence is restrictive annuloplasty, which often involves reshaping or remodeling the annulus by attaching a prosthetic annuloplasty repair segment or ring thereto. A "remodeling" annuloplasty ring typically has an inner core that resists conforming to the native annulus shape and instead forces the annulus to conform to it. Remodeling annuloplasty bands or rings are "generally rigid" or "semi-rigid" in that they will resist distortion when subjected to the stress imparted thereon by the mitral valve annulus of an operating human heart. In this sense, "distortion" means substantial permanent deformation from a predetermined or manufactured shape *(e.g.,* the ring or ring will tend to return to its preset shape in use). A typical remodeling annuloplasty ring comprises an inner substrate or core of a metal such as a rod or multiple bands of stainless steel or titanium covered with a biocompatible fabric or cloth and perhaps silicone to allow the ring to be sutured to the fibrous annulus tissue. The Physio^{®} ring from Edwards Lifesciences of Irvine, CA is a closed "semi-rigid" ring because it offers selective flexibility at the posterior section while preserving the remodeling effect through a rigid anterior section. The newer Physio II^{®} ring from Edwards Lifesciences also features up and down curves to better fit the nonplanar contours of the mitral annulus. Various other rings have posterior bows, e.g., U.S. Patent Nos. 6,805,710 and 6,858,039, 7,959,673, or other three-dimensional configurations. US 2003/0083742 A1 describes a device including a support member configured for attachment to the heart valve annulus, a post extending from the support member away from the plane of the annulus and a connector coupled with the post and configured for attachment to at least one of the leaflets.

Other than revascularization, the treatment for ischemic mitral regurgitation remains controversial. Restrictive annuloplasty alone may alleviate the regurgitation in the short-term, but the long-term results are poor. This is due to continued expansion/remodeling of the ventricle, which is not addressed by simple reductive annuloplasty. As such, there has been a movement toward valve replacement in IMR patients; however, replacement in these patients has been shown to have higher perioperative mortality. The elimination of MR may be more durable in replacement patients as prosthetic valves are not affected by ventricular geometry, but long-term survival is still poor as the ventricle is not addressed and may continue to remodel to a more advanced stage of heart failure.

Work pioneered by Irving Kron and colleagues has shown that relocating/suspending the papillary muscles toward the mitral annulus using sutures, in conjunction with a non-restrictive annuloplasty, creates a durable and long-lasting repair. These methods have been widely studied and reported on by Kron and colleagues as well as many others, with promising results in terms of reduced MR reoccurrence and improved mortality. However, despite the very promising results using these seemingly simple techniques, they have not been widely adopted, perhaps because it is difficult to know how much to relocate the papillary muscles. As with all open mitral procedures, these methods are performed on an arrested, depressurized heart. As such, the geometry of the mitral valve, subvalvular apparatus, and ventricle, are all in a completely different state compared to when the heart is a pressurized, actively contracting organ.

Despite numerous therapeutic solutions presently available or proposed in the past, there is a need for a better repair procedure for mitral regurgitation caused by ischemia or dilated cardiomyopathy.

### SUMMARY

The present disclosure provides an annuloplasty ring and subvalvular attachment for repair of mitral and tricuspid valves experiencing regurgitation due to diseases of the heart muscle such as ischemia or cardiomyopathy. The repair involves implanting an annuloplasty ring at the annulus to which tethers are attached connected to subvalvular structures such as the papillary muscles. The annuloplasty ring and tether adjustment system incorporate features for papillary muscle relocation that can be implanted on-pump and adjusted off-pump for an optimal result. The repair is performed on an arrested heart, but then the final length and tension of the papillary muscle relocation tethers are fine-tuned and ultimately locked while the heart is beating. Visualization helps determine the optimal tether length.

In one example, an annuloplasty ring for repair of mitral and tricuspid valves including an inner core covered by an outer suture-permeable interface. The inner core defines a closed peripheral shape surrounding an orifice and at least one outwardly-projecting boss configured to provide a suture anchor, such as having a pair of apertures. A plurality of anchoring sutures pass through the interface and secure the annuloplasty ring to a native heart valve annulus, while at least one tethering suture ties to the suture anchor and extends to attach to structure in a subvalvular chamber below the native heart valve annulus. The length of the tethering suture is adjusted while the heart is beating using a tensioning system to ensure proper valve functioning.

According to the present invention as defined by appended claim 1, an annuloplasty ring and tether adjustment system comprises an annuloplasty ring including an inner core covered by an outer suture-permeable interface. The inner core defines a closed peripheral shape surrounding an orifice and the interface closely surrounds the inner core and has a matching shape. The inner core further defines at least one outwardly-projecting boss configured to provide a suture anchor. A plurality of anchoring sutures adapted to pass through the interface secure the annuloplasty ring to a native heart valve annulus. At least one tethering suture is configured to loop through subvalvular structure in a chamber below the native heart valve annulus and tie off thereto. The tethering suture has a length such that a pair of free ends extends from the subvalvular structure through the outwardly-projecting boss on the inner core and out of the body. A tether adjusting mechanism includes at least one tubular arm having a length sufficient to extend from the native heart valve annulus through an access incision in the heart and out of the body. The pair of free ends of the at least one tethering suture pass through the at least one tubular arm, and the pair of free ends has a first end and a second end. A tensioner receives the second end, and the at least one tubular arm has sufficient column strength such that tension on the second end caused by the tensioner decreases the distance between the subvalvular structure and the annuloplasty ring, at which point the first and second end are configured to tie off to the outwardly-projecting boss.

The annuloplasty ring and tether adjustment system preferably has at least two outwardly-projecting bosses spaced apart around the inner core, and in one example there are three outwardly-projecting bosses spaced apart around the inner core, two of which are more closely spaced to each other than from a third. The suture-permeable interface may have a silicone inner portion surrounded by a fabric cover.

The annuloplasty ring may be adapted to be implanted at the mitral annulus, such that the inner core peripheral shape is a rounded D-shape with an anterior segment on a substantially straight side opposite a more curved posterior segment, and wherein the at least one outwardly-projecting boss is located in the posterior segment. The inner core may define a major axis across a long dimension perpendicular to a minor axis, wherein the anterior segment and the posterior segment are on opposite sides of the major axis, and wherein there are at least two outwardly-projecting bosses on opposite sides of the minor axis and located in the posterior segment. In one example, there are only two outwardly-projecting bosses, one on each side of the minor axis and two tethering sutures.

In the annuloplasty ring and tether adjustment system, the tether adjusting mechanism may have a tubular arm for each tethering suture, and further include a common access sheath through which the tubular arms extend. The at least one outwardly-projecting boss may comprise a rounded rectangular projection through which a pair of identical apertures extend, or a T-shaped projection having rounded ends.

An exemplary annuloplasty ring and tether adjustment system disclosed herein (not part of the invention) comprises an annuloplasty ring including an inner core covered by an outer suture-permeable interface. The inner core defines a closed peripheral shape surrounding an orifice and the interface closely surrounds the inner core and has a matching shape. The inner core defines at least one outwardly-projecting boss configured to provide a suture anchor. A plurality of anchoring sutures are adapted to pass through the interface and secure the annuloplasty ring to a native heart valve annulus. Finally, at least one tethering suture is tied to the outwardly-projecting boss and has a length sufficient to extend from the outwardly-projecting boss to attach to structure in a subvalvular chamber below the native heart valve annulus.

There may be at least two outwardly-projecting bosses spaced apart around the inner core, and in one example there are three outwardly-projecting bosses spaced apart around the inner core, two of which are more closely spaced to each other than from a third. The suture-permeable interface may comprise a silicone inner portion surrounded by a fabric cover. In one example, the annuloplasty ring is adapted to be implanted at the mitral annulus, and the inner core peripheral shape is a rounded D-shape with an anterior segment on a substantially straight side opposite a more curved posterior segment, and wherein the at least one outwardly-projecting boss is located in the posterior segment. Furthermore, the inner core may define a major axis across a long dimension perpendicular to a minor axis, wherein the anterior segment and the posterior segment on opposite sides of the major axis, and wherein there are at least two outwardly-projecting bosses on opposite sides of the minor axis and located in the posterior segment. The at least one outwardly-projecting boss may comprise a rounded rectangular projection through which a pair of identical apertures extend, or a T-shaped projection having rounded ends.

A method for implanting an annuloplasty ring at a native heart valve annulus and adjusting subvalvular structure below the native heart valve annulus (not part of the invention), comprises:
stopping a patient's heart and providing an access pathway to a native heart valve annulus, including an access incision in the heart leading to the native heart valve annulus;
looping a plurality of anchoring sutures through the heart valve annulus at spaced locations and retracting free ends of the anchoring sutures out of the body;
looping at least one tethering suture through a subvalvular structure below the heart valve annulus and retracting free ends of the tethering suture out of the body;
passing one end of each of the pairs of free ends of the anchoring sutures through a peripheral suture-permeable interface of an annuloplasty ring, the annuloplasty ring including an inner core defining a closed peripheral shape surrounding an orifice and the interface closely surrounding the inner core and having a matching shape, the inner core defining at least one outwardly-projecting boss configured to provide a suture anchor;
passing both free ends of the at least one tethering suture upward through the outwardly-projecting boss and retracting the free ends of the at least one tethering suture out of the body;
advancing the annuloplasty ring to the heart valve annulus along the anchoring sutures;
tying off the anchoring sutures on a proximal side of the annuloplasty ring;
sealing the access incision around the free ends of the at least one tethering suture;
restarting the patient's heart;
externally visualizing blood flow through the heart valve annulus;
adjusting the tension on one of the free ends of the at least one tethering suture;
securing the free ends of the at least one tethering suture on the proximal side of the annuloplasty ring at the boss, and severing the free ends of the at least one tethering suture; and
withdrawing the at least one tethering suture from the body and closing up the access pathway.

In the method, after the step of advancing the annuloplasty ring to the heart valve annulus, the method may include advancing a tubular arm down each pair of free ends of tethering sutures from outside the body until the tubular arm contacts the annuloplasty ring, and wherein the step of adjusting the tension on one of the free ends of the at least one tethering suture places the tubular arm in compression. There may be at least two outwardly-projecting bosses spaced apart around the inner core and at least two tethering sutures. The tubular arms may pass through a common access sheath, and the access incision is sealed around the common access sheath.

In the method, each pair of free ends may have a first end and a second end, and further including a tensioner that receives the second end, the at least one tubular arm having sufficient column strength such that tension on the second end caused by the tensioner decreases the distance between the subvalvular structure and the annuloplasty ring, at which point the first and second end are configured to tie off to the outwardly-projecting boss.

In the method, the at least one outwardly-projecting boss may comprise a rounded rectangular projection through which a pair of identical apertures extend, or a T-shaped projection having rounded ends.

All methods disclosed herein also encompass simulations of the methods, for example, for training; testing; demonstration; or device or procedure development. Methods for treating a patient can include simulating treatment on a simulated human or non-human patient, for example, an anthropomorphic ghost. Examples of suitable simulated patients can include both an entire body, any portion of a body, or at least a portion of an organ, for example, a heart. The simulations can be physical, virtual, or any combination thereof. Examples of physical simulations can include any combination of natural or manufactured whole human or animal cadavers, portions thereof, or cadaver organs. Virtual simulations can include any combination of virtual reality, projections onto a screen or on at least a portion of a physical simulation, or other *in silico* elements. Some simulations can include non-visual elements, for example, auditory, tactile, or olfactory stimuli.

A further understanding of the nature and advantages will become apparent by reference to the remaining portions of the specification and drawings.

### BRIEF DESCRIPTION OF THE DRAWINGS

Features and advantages will become appreciated as the same become better understood with reference to the specification, claims, and appended drawings wherein:
Figure 1A is an atrial plan view of a mitral valve and leaflets indicating common nomenclature initials for regular anatomical features;
Figure 1B is a schematic view of the three-dimensional shape of the mitral annulus with several anatomical landmarks indicated;
Figure 2A is a schematic perspective view from the atrial side of a healthy mitral valve and a sectional view of the subvalvular anatomy of the left ventricle, and Figure 2B is the same view for a heart suffering from ischemia in the left ventricle which causes distention and regurgitation;
Figure 3 is a sectional view of an ischemic left ventricle after implantation of a corrective annuloplasty ring and subvalvular structure disclosed herein;
Figures 4A-4E are orthogonal and sectional views of an exemplary annuloplasty ring of the present application;
Figures 5A-5D are plan views of different versions of an inner core of the exemplary annuloplasty ring;
Figures 6A-6D show a sequence of certain steps for implanting the corrective annuloplasty ring and subvalvular structure;
Figure 7 is a schematic view of a tether adjustment system for the corrective annuloplasty ring and subvalvular structure in use; and
Figures 8A-8F show a sequence of several final steps for securing the subvalvular structure.

### DETAILED DESCRIPTION

The right ventricle RV and left ventricle LV are separated from the right atrium RA and left atrium LA, respectively, by the tricuspid valve TV and mitral valve MV; *e.g.,* the atrioventricular valves. Though correction of the mitral annulus is the primary focus of the present application, it should be understood that certain characteristics of the annuloplasty rings described herein may equally be used to treat the tricuspid valve TV, and thus the claims should not be constrained to the mitral ring unless expressly limited.

The application discloses an annuloplasty ring that incorporates bosses for tying off sutures that can be placed through papillary muscles (PM), along with a tether adjustment system for tensioning the PM sutures on a beating heart. The ring and papillary muscle sutures would be implanted on an on-pump, arrested heart. The shaft of the tether adjustment system could be passed through a small opening in the wall of the atrium and sealed with purse-string sutures to form a hemostatic seal such that the heart can be weaned off bypass for final adjustment of the PM suture lengths.

The term "axis" in reference to the illustrated annuloplasty rings, and other non-circular or non-planar rings, refers to a line generally through the centroid of the ring periphery when viewed in plan view. "Axial" or the direction of the "axis" can also be viewed as being parallel to the average direction of blood flow within the valve orifice and thus within the ring when implanted therein. Stated another way, an implanted mitral ring orients about a central flow axis aligned along an average direction of blood flow through the mitral annulus from the left atrium to the left ventricle. The plan views of the annuloplasty rings illustrated herein are as looking from the atrial side in the direction of blood flow. For the purpose of orientation, therefore, the atrial side of the ring is up in the ventricular site is down.

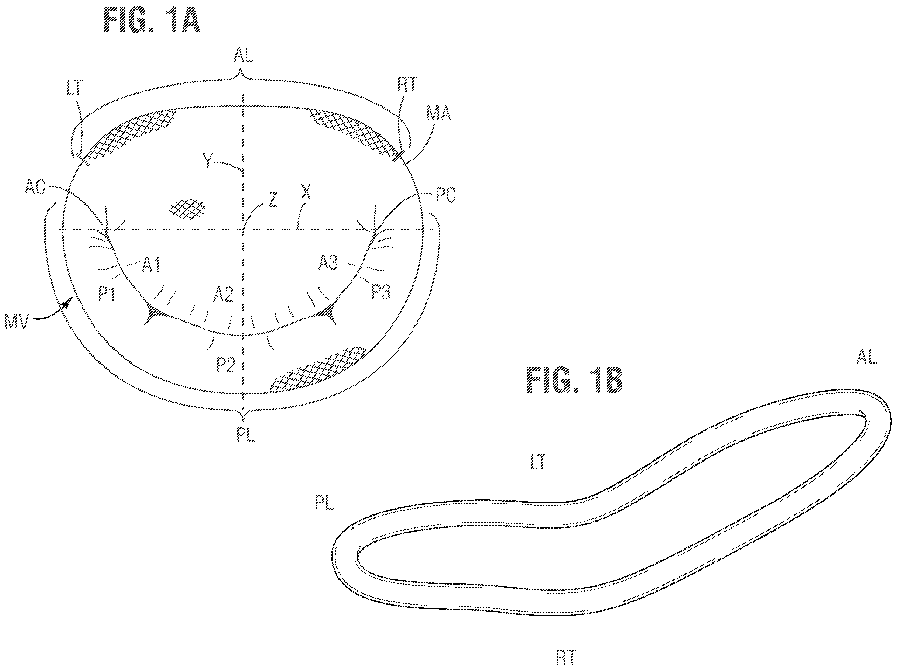

Figure 1A is a schematic perspective view from the atrial side of a mitral valve MV with posterior being down and anterior being up. The mitral valve MV primarily comprises a pair of coapting leaflets - an anterior leaflet AL and a posterior leaflet PL - secured around their outer edges to a fibrous mitral annulus MA. The surrounding mitral annulus MA is often described as D-shaped with a somewhat straighter side adjacent the anterior leaflet AL and a more rounded or convex side adjacent the posterior leaflet PL. The mitral annulus MA is typically viewed as having a major axis X that intersects both the first and third posterior scallops P1 and P3, approximately at the commissures AC, PC, and a minor axis Y that intersects and generally bisects the middle posterior scallop P2. A central flow axis Z is arbitrarily defined at the intersection of the major and minor axes X, Y.

The leaflets are shaped such that the line of coaptation resembles a smile that approximately parallels the posterior aspect of the mitral annulus MA. The anterior leaflet AL spans a smaller peripheral aspect around the mitral annulus MA than the posterior leaflet PL, but the anterior leaflet AL has a convex free edge that extends farther into the orifice defined by the mitral annulus MA. The posterior leaflet PL, on the other hand, has a generally concave free edge. Two commissures - an anterior commissure AC and a posterior commissure PC - generally defined the intersection of the line of coaptation between the two leaflets AL, PL and the mitral annulus MA. The posterior leaflet is divided into three scallops or cusps, sometimes identified as P1, P2, and P3, starting from the anterior commissure AC and continuing in a counterclockwise direction to the posterior commissure PC. Per convention, a major axis of the mitral annulus intersects both the first and third posterior scallops P1 and P3, approximately at the commissures AC, PC, and a minor axis intersects and generally bisects the middle posterior scallop P2. The anterior leaflet also features scallops or regions labeled A1, A2, and A3 as indicated in Figure 1A.

As illustrated, the mitral annulus has a kidney or rounded D-shape around its periphery. The mitral anterior leaflet AL attaches to a somewhat straight anterior fibrous portion of the mitral annulus, which makes up about one-third of the total mitral annulus circumference. The anterior fibrous annulus, the two ends of which are called the fibrous left and right trigones LT, RT, forms part of the central fibrous skeleton of the heart. The arcuate muscular portion of the mitral annulus constitutes the remainder of the mitral annulus, and the posterior leaflet PL attaches thereto. The anterior commissure AC and the posterior commissure PC are located just posterior to each fibrous trigone.

Figure 1B is a schematic view of the three-dimensional shape of the mitral annulus with several anatomical landmarks indicated. In particular, the anterior leaflet AL is separated from the posterior leaflet PL by the left and right trigones LT, RT. The three-dimensional shape is somewhat like a saddle, with the trigones LT, RT in a valley and the leaflets AL, PL rising up.

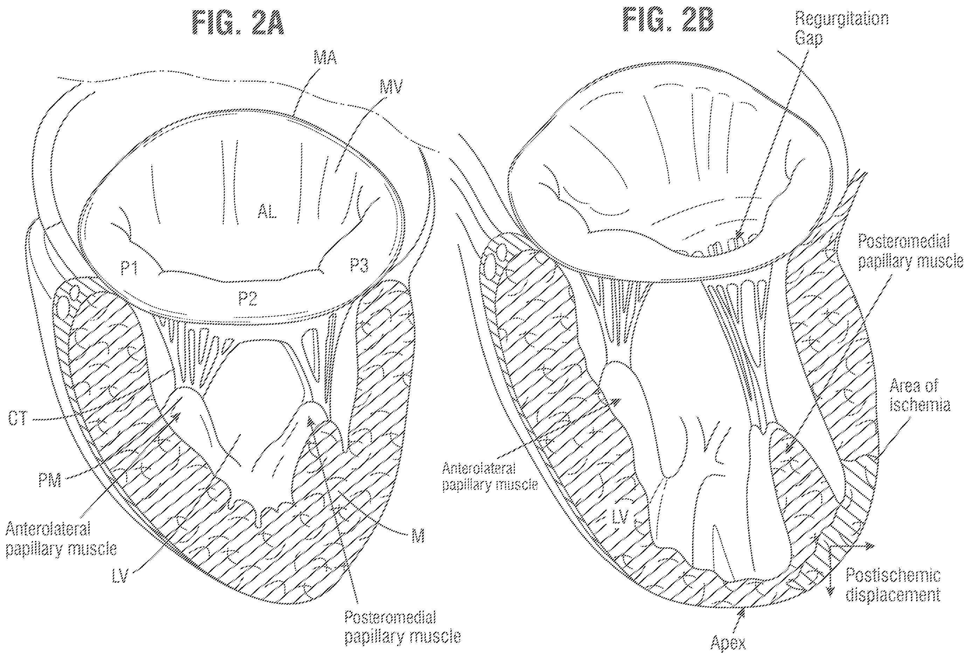

Figure 2A is a schematic perspective view from the atrial side of a healthy mitral valve and a sectional view of the subvalvular anatomy of the left ventricle. The inner edges of the anterior and posterior leaflets AL, PL connect to string-like chordae tendineae CT that extend down into the left ventricle LV and are tethered at papillary muscles PM that extend upward from the muscular myocardium M defining the left ventricular cavity. There are two papillary muscles: an anterolateral and a posteriomedial muscle. The chordae tendineae extending upward from each of the papillary muscles connects to parts of both of the leaflets AL, PL. As the main outlet pumping chamber of the heart, the myocardium M contracts in systole which reduces tension in the chordae tendineae CT and permits the leaflets AL, PL to come together or coapt. Contraction of the volume within the left ventricle LV sends blood out through the aortic valve (not shown) and into the body. In diastole, the myocardium M expands outward pulling the chordae tendineae CT down in tension to help open the leaflets AL, PL and pulling blood through the mitral valve MV to fill the cavity of the left ventricle LV.

Figure 2B is the same view for a heart suffering from ischemia in the left ventricle which causes distention and regurgitation. The ischemic area is indicated toward the lower apex of the ventricle and tends to cause distension of the myocardium at that location, resulting in a regurgitation gap between the mitral valve leaflets. A similar dynamic occurs with other diseases of the heart muscle such as cardiomyopathy.

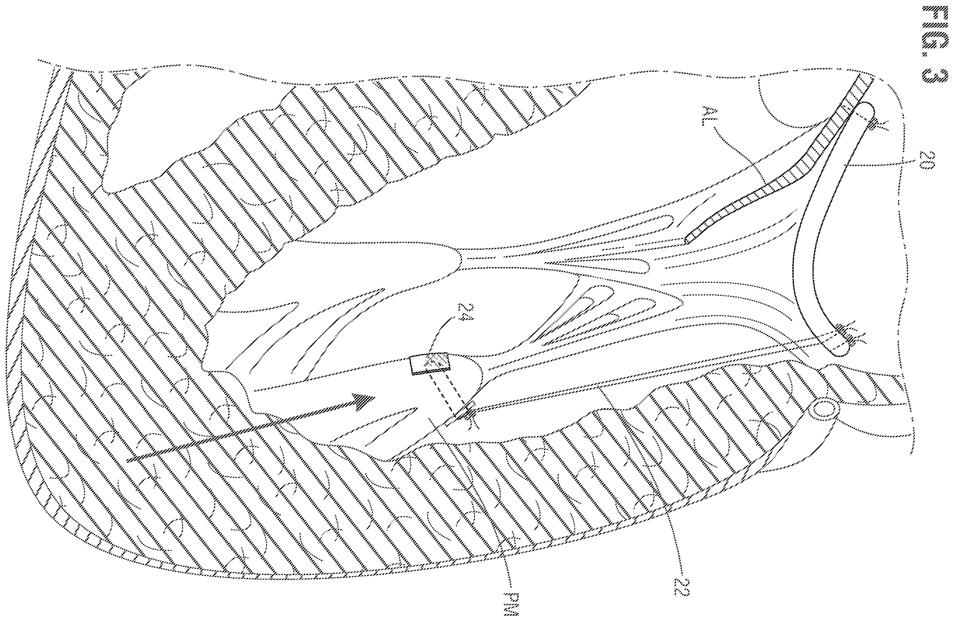

Figure 3 is a sectional view of an ischemic left ventricle after implantation of a corrective annuloplasty ring 20 and at least one subvalvular suture 22, such as a papillary muscle (PM) suture. The PM suture 22 is passed through one of the papillary muscles, possibly with a pledget 24 on both sides as shown, and then the two free lengths of the suture are tied together on the side where they extend toward the annulus. Prior to securing the PM suture 22 to the annuloplasty ring 20, and as explained in more detail below, this allows the tension on the PM to be adjusted by only one free length of the suture and subsequent tying of the two free lengths of the suture. Tension is created in the one or more PM sutures 22 to pull the papillary muscles upward toward the anchored annuloplasty ring 20, as shown by the large arrow, allowing the mitral valve leaflets AL, PL to better coapt, thus reducing the problematic regurgitation.

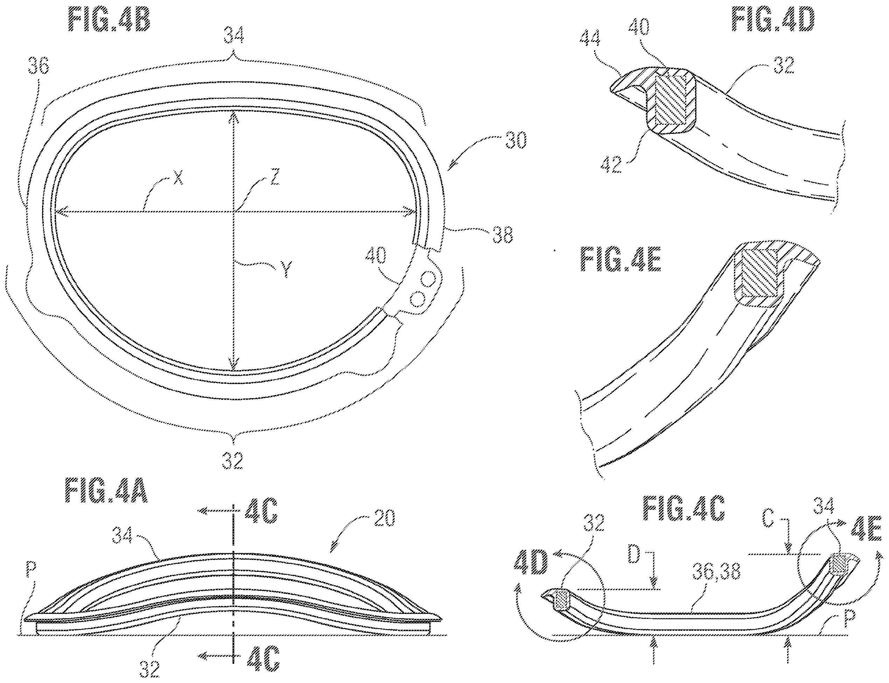

Figures 4A-4E are orthogonal and sectional views of an exemplary annuloplasty ring 30 of the present application. The annuloplasty ring 30 has a posterior segment 32 opposite an anterior segment 34, with side segments 36, 38 in between. Some nomenclature has the posterior segment 32 extending roughly around the posterior leaflet PL (Figure 1A), extending between the leaflet commissures AC, PC, but generally the posterior segment 32 is bisected by a minor axis Y and extends at least around the middle posterior scallop P2 of the posterior leaflet PL. A major axis X and a minor axis **Y** are indicated which, when the annuloplasty ring 30 is implanted, coincide with the same axes of the native mitral annulus, such that blood will flow into the page generally parallel to a flow axis Z through the middle of the ring. The plan view shape of the annuloplasty ring 30 is kidney or rounded D-shaped so as to conform to the peripheral shape of the typical mitral annulus.

The annuloplasty ring 30 may be three-dimensional with an upward bow in the posterior segment 32 as well as an upward bow in the anterior segment 34, as seen in Figures 4A and 4C. Preferably the anterior segment 34 bows upward a distance C from a reference plane P more than the distance D of an upward bow of the anterior segment 34 above the reference plane P. The side segments 36, 38 may lie in the reference plane P such that the ring 30 is partially planar with the two opposite upward bows, to form somewhat of a saddle shape so as to better conform to the native mitral annulus, as depicted in Figure 1B. The shape of the ring 30 is similar to that of the Physio II^{®} annuloplasty ring available from Edwards Lifesciences of Irvine, CA.

Figures 4C and 4D are sectional views of the annuloplasty ring 30 taken along corresponding section line 4C-4C in Figure 3A. In a preferred example, the ring construction includes a relatively rigid inner core 40 surrounded by a suture-permeable interface. In the illustrated example, the inner core 40 is solid and rectangular, with a greater axial dimension than radial dimension. As will be explained below, the material of the inner core 40 is an elastic-plastic material such as stainless steel or titanium alloy which permanently deforms after reaching a yield stress. Rather than an elastic plastic metal, an elastic-plastic polymer such as polyamide (*e.g.,* nylon), polyacetal (*e.g.,* DELRIN^{®} polyoxymethylene, DuPont, Wilmington, Delaware) or the like may also be utilized. The annuloplasty ring 30 is thus a remodeling ring.

The suture-permeable interface may include an elastomeric sleeve 42 closely surrounding the core and a fabric outer cover (not shown), for example, a polyethylene terephthalate (PET) fabric cover. In the preferred example the elastomeric sleeve 42, which may be silicone rubber, is generally tubular and molded to have a radially outwardly-extending flange 44 to facilitate suturing of the ring 30 to the mitral annulus. The ring 30 may be secured with sutures, staples, or other such devices to an inside fibrous ledge of the mitral annulus. In a typical procedure, the surgeon anchors an array of sutures through the annulus and then threads them through corresponding locations around the interface on the outside of the ring 30. The ring is parachuted down the suture array to be seated at the annulus before tying off the sutures.

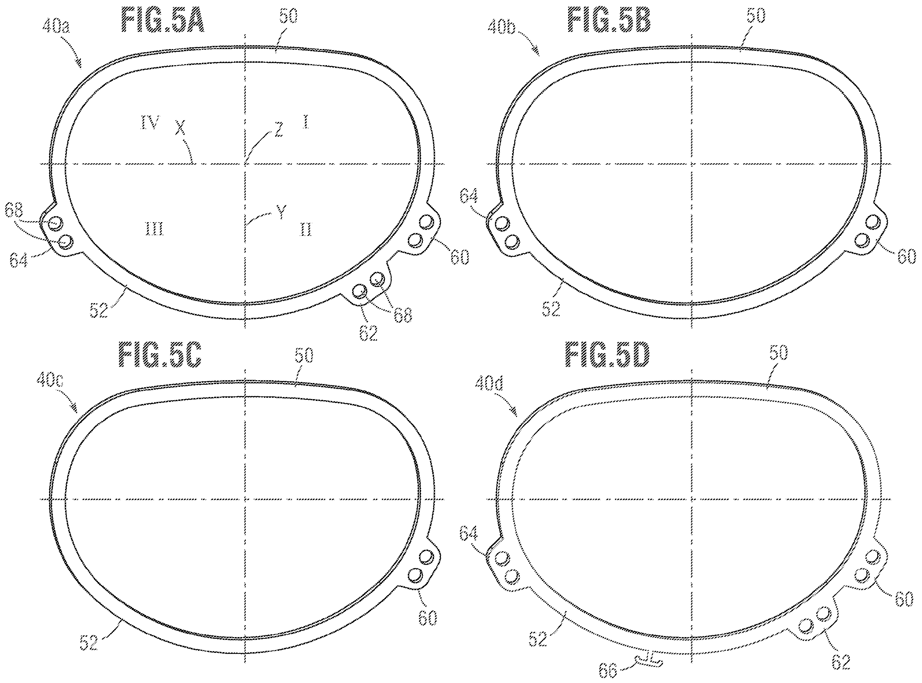

Figures 5A-5D are plan views of different versions of the inner core 40 of the exemplary annuloplasty ring. The inner core 40 is a closed shape with a generally straight anterior segment 50 opposite a more curved posterior segment 52, such that the overall peripheral shape is a rounded D-shape. In each case, a major axis X and a minor axis Y are indicated which, when the annuloplasty ring 30 is implanted, coincide with the same axes of the native mitral annulus. These plan views of the inner core 40 are as looking from an atrial side of the annuloplasty ring 30, such that blood will flow into the page generally parallel to a flow axis Z through the middle of the ring.

As indicated in Figures 4D and 4E, the inner core has a generally rectangular radial cross-section. The magnitude of the inner core cross-section is preferably constant around the periphery, though relatively thick and thin portions may be provided to inhibit or enhance flexibility in different areas.

Each of the inner cores 40 shown in Figures 5A-5D has at least one radially outward boss which serves to anchor a papillary muscle (PM) tether, as will be shown. To help locate the bosses, four quadrants I, II, III, IV are labeled within the inner core 40 delineated by the major and minor axes X, Y and starting at the upper right and going clockwise. The first and fourth quadrants I, IV above the major axis X generally coincide with the anterior segment 50, and the second and third quadrants II, III below the major axis X generally coincide with the posterior segment 52.

Each of the bosses 60, 62, 64 is formed by a generally rectangular projection with rounded corners and has two through holes or apertures 68 formed therein. The apertures 68 provide passageways for sutures when anchoring the annuloplasty ring 30 to subvalvular structures. Apair of separated apertures 68 enables the suture to be tied off and thus anchored to the ring. It should also be understood that rather than simple holes through the bosses, clamping structure may also be incorporated which enables anchoring of each of the tethering sutures without tying knots. For instance, SUTRAFIX^{®} fasteners (Edwards Lifesciences, Irvine, California) could be deployed on the tethering sutures 72 relatively easily upon proper tensioning. Likewise, crush sleeves, cleats, wedges, screws, and other common mechanisms for securing two cords could be used. However, this would add some complexity and increase the cost of the device considerably.

The inner core 40a of Figure 5A has three outward bosses 60, 62, 64, all below the major axis X in the posterior segment 52. First and second bosses 60, 62 extend outward from the inner core 40a and the second quadrant II, while a third boss 64 extends outward in the third quadrant III. The Hamburg group led by Evaldas Girdauskas has shown particularly good results by relocating the main head of the anterior-lateral PM and 2 of the 3 heads of the posterior-medial PM with sutures between the muscles and the native mitral annulus *(see* "Minimally invasive mitral valve repair for functional mitral regurgitation," European Journal of Cardio-Thoracic Surgery, Volume 55, Issue Supplement 1, June 2019, Pages i17-i25). The Hamburg group also places the sutures around the P1 and P3 scallops of the mitral valve. Therefore, the inner core 40a of Figure 5A has two bosses on the posterior-medial side of the ring core and a single boss on the anterior-lateral side.

Figure 5B shows an inner core 40b with just two bosses: a first boss 60 extending outward in the second quadrant II, and a second boss 64 extending outward in the third quadrant III. The core 40c in Figure 5C shows just a single boss 60 extending outward in the second quadrant II. Finally, Figure 5D illustrates a core 40d with more than three, specifically four bosses. First and second bosses 60, 62 project outward in the second quadrant II, and third and fourth bosses 64, 66 project outward in the third quadrant III.

The fourth boss 66 is shown as a variation from the others. Namely, the boss 66 comprises an outwardly-directed T-shaped projection having rounded ends that curl back inward toward adjacent portions of the core 40d. Tethering sutures can be passed on both sides of the boss 66 and tied off, much like the pair of apertures formed in the other bosses 60, 62, 64. As such, all of the bosses 60, 62, 64, 66 are configured as suture anchors. Of course, other outwardly-directed structures that enable a suture to be tied off are contemplated, and the disclosure should not be considered limited to the illustrated examples.

The location of the outward bosses depends on how the ring 30 will be used to reshape the left ventricle, which in turn depends on the pathological condition. For example, for the ischemia illustrated in Figure 2B, just a single boss 60 as seen in Figure 5C may be sufficient to tether the ring to the posterior-medial (posteriomedial) papillary muscle. Alternatively, a ring 30 having the inner core 40a of Figure 5A may be preferred so that two tethers 60, 62 may be attached to the different heads of the posteriomedial papillary muscle, while the third tether 64 attaches to the anterior-lateral (anteriolateral) papillary muscle. Other pathological conditions may dictate other arrangements. It should be understood that most of the pathological conditions lead to left ventricular distention in the outer myocardium, and thus tethers are preferably attached around the posterior segment 32, or below the major axis X, which is the portion of the ring implanted adjacent the myocardium.

Other researchers have relocated a single PM head on each side, and some have even only relocated a single PM. Likewise, there is some variation in where the sutures from the PM(s) are located on the annulus, with some investigators placing them closer to the fibrous trigones or commissures. As such, any number of bosses and any location of the bosses is possible and is not restricted to the examples shown here.

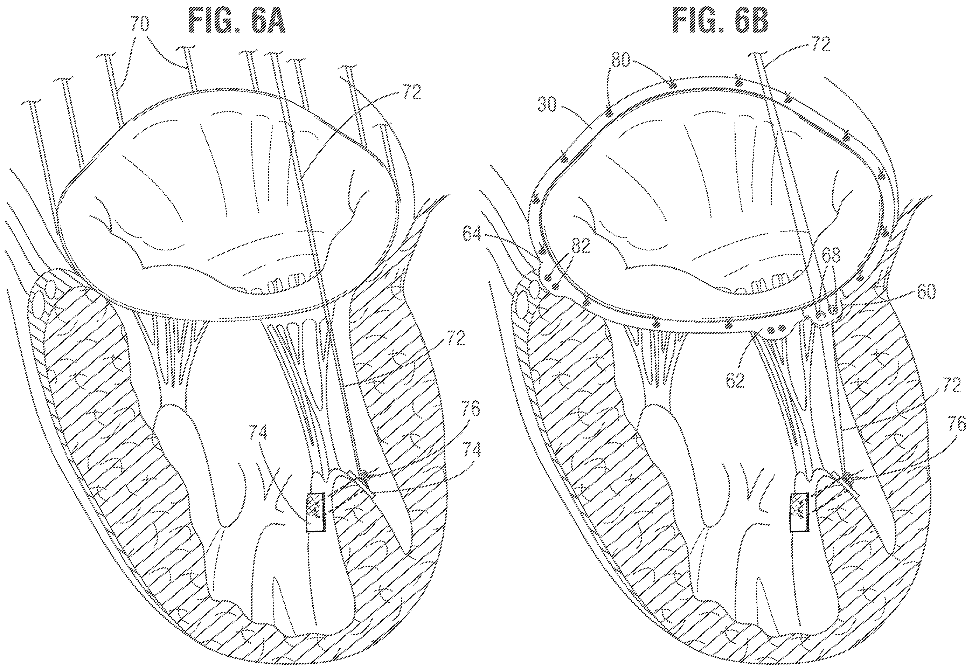

Figures 6A-6D show a sequence of certain steps for implanting the corrective annuloplasty ring 30 and subvalvular structural tethers. While the patient is on cardiopulmonary bypass in the heart is stopped, anchoring sutures 70 are looped around the annulus at spaced intervals and retracted out of the body in an orderly fashion, as seen in Figure 6A. Typically, a suture organizer is utilized. One or more tethering sutures 72 are installed within the left ventricle LV, typically through the heads of one or more papillary muscles, as shown. As mentioned, patches or pledgets 74 are typically used on both sides of the papillary muscle to reinforce and prevent the tethering sutures 72 from pulling through the muscle. Each of the tethering sutures 72 are then tied off at a knot 76 on the side of the muscle where the tethering suture extends upward and out of the body.

Figure 6B is a snapshot after the annuloplasty ring 30 has been parachuted down the array of anchoring sutures 70 into contact with the mitral annulus. Each of the anchoring sutures 70 is then tied off above the ring 30 at knots 80. This securely anchors the annuloplasty ring 30 to the mitral annulus MA. Although the annuloplasty ring 30 has the inner core 40a from Figure 5A with three outward bosses, the tethering sutures 72 are only shown passing through the first boss 60 at which the outer fabric cover of the ring has been cut away. The other two bosses 62, 64 are shown with two indicator dots 82 each which help the surgeon locate the apertures 68 through the bosses. The indicator dots 82 may be imprinted on the outer fabric cover, or may be visualized using an individual suture at that location.

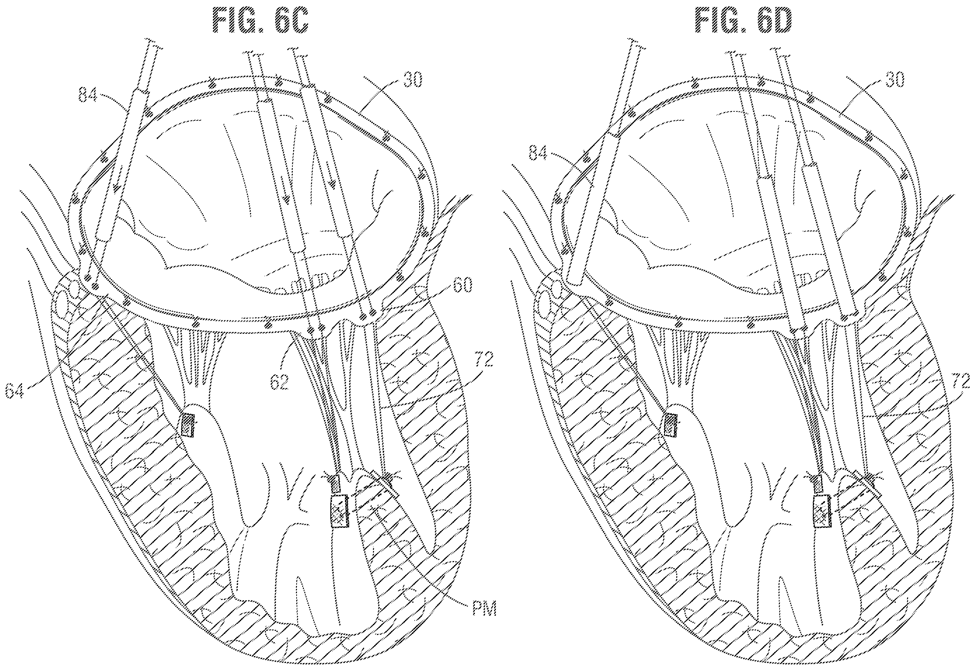

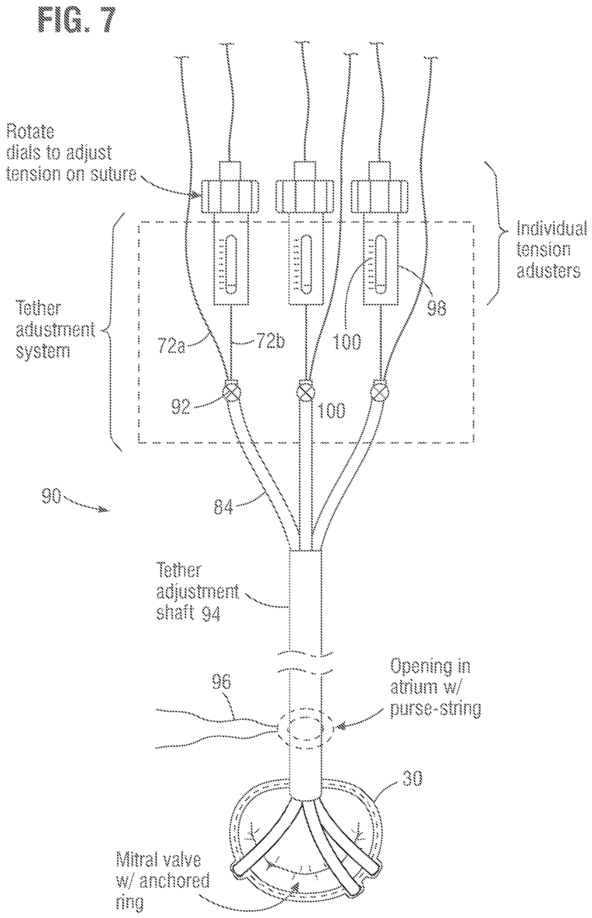

Figure 6C shows three tethering sutures 72 passing through the three outward bosses 60, 62, 64 around the ring 30, and advancement of three tubular arms 84 of a tether adjustment system 90, as seen in Figure 7. The tethering sutures 72 can be very simply pulled through the tubes 84 using snares. As mentioned above, the tethering sutures 72 passing through the first two bosses 60, 62 have been secured to different heads of the posteriomedial papillary muscle, while the tethering sutures 72 passing through the third boss 64 attaches to the head of the anteriolateral papillary muscle.

Subsequently, Figure 6D shows the three arms 84 having been advanced all the way into contact with the proximal side of the annuloplasty ring 30. As will be explained, contact of the arms 84 against the ring 30 provides a brace to enable adjustment of each of the tether sutures 72. That is, the tubular arms 84 will resist the tension applied to the PM relocation sutures 72 by carrying a compressive load, but will be flexible to allow for positioning and movement. It is this stage that the patient is weaned off cardiopulmonary bypass and the heart restarted, but first a description of the tether adjustment system 90 is appropriate.

Figure 7 is a schematic view of the tether adjustment system 90 for tethering sutures 72. The three tubular arms 84 are shown advanced into contact with the bosses on the annuloplasty ring 30. It should be noted that the individual tubular arms 84 are each outfitted with a hemostatic valve (schematically indicated at 92) to prevent blood from freely flowing through it when off-pump adjustments are being made. Each of the three arms 84 emerges from a distal end of an inner lumen of a common tether adjustment shaft 94. The shaft 94 extends from outside of the body into proximity with the mitral valve, such as into the left atrium. One access route is through a small hole formed in the left atrial wall, and a hemostatic seal formed using a purse-string suture 96 as shown. With the purse-string suture 96 tightened around the shaft 94, the heart may be restarted without risk of leakage around the shaft.

The tether adjustment shaft 94 extends proximally and the three arms 84 diverge at some point and the individual tethering sutures 72 are then separated. The lumen for each arm 84 is separate from end to end to allow for independent routing, tensioning, and tying of each PM relocation suture 72.

One free end 72a of each pair of tethering sutures 72 remains slack, while the second free end 72b is held taut and passes through a tension adjuster 98. The dashed outline indicates a housing of some sort which maintains rigidity between each of the tension adjusters 98 and the arms 84 to allow the tension adjusters to increase or decrease tension on each of the tethering sutures 72 relative to their eventual point of anchor within the left ventricle. In the illustrated example, the tension adjusters 98 have rotating dials which gradually adjust the tension on the corresponding suture, and a gauge 100 indicating the absolute tensile force on each may be used. Alternatively, the gauge 100 may be calibrated to indicate distance. Of course, sliders or other tension adjusters can be utilized. While the patient's heart is beating and the mitral valve is subjected to external visualization, such as fluoroscopy, tension on each of the tethering sutures 72 may be adjusted in coordination until regurgitation through the mitral valve is minimized.

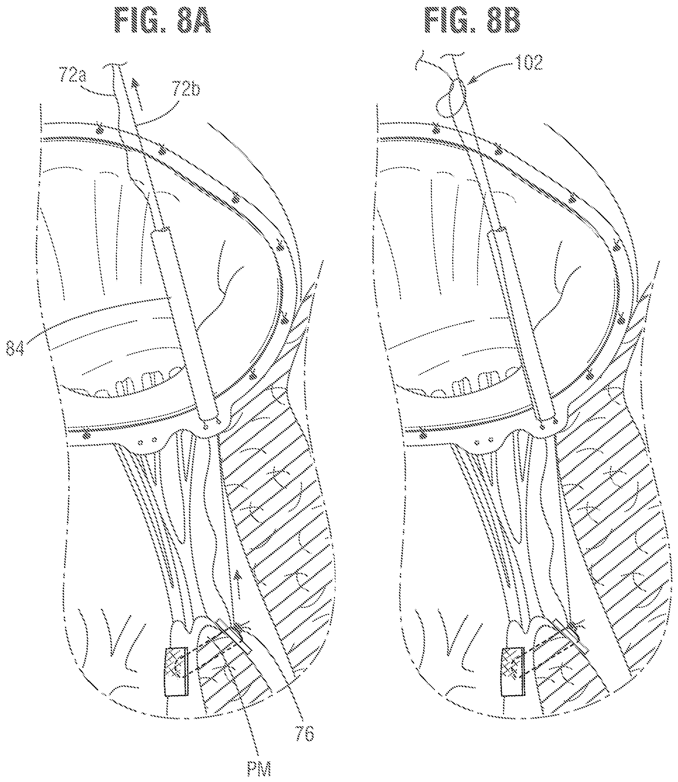

Figures 8A-8E show a sequence of several final steps for securing the subvalvular structure, though only one of the tethering sutures 72 and corresponding arm 84 is shown. The first free end 72a is shown slack, while the second free end 72b is taut. The movement arrows in Figure 8A indicate tension being pulled on the taut free end 72b, which translates to proximal movement of the relevant papillary muscle PM. As mentioned, the one or more tensioned adjusters 98 are operated while blood flow through the mitral valve MV is visualized until no or an acceptable level of regurgitation is seen. At this point, a constant tension on the second free end 72b is maintained while the tensioning sutures 72 are tied off against the annuloplasty ring 30.

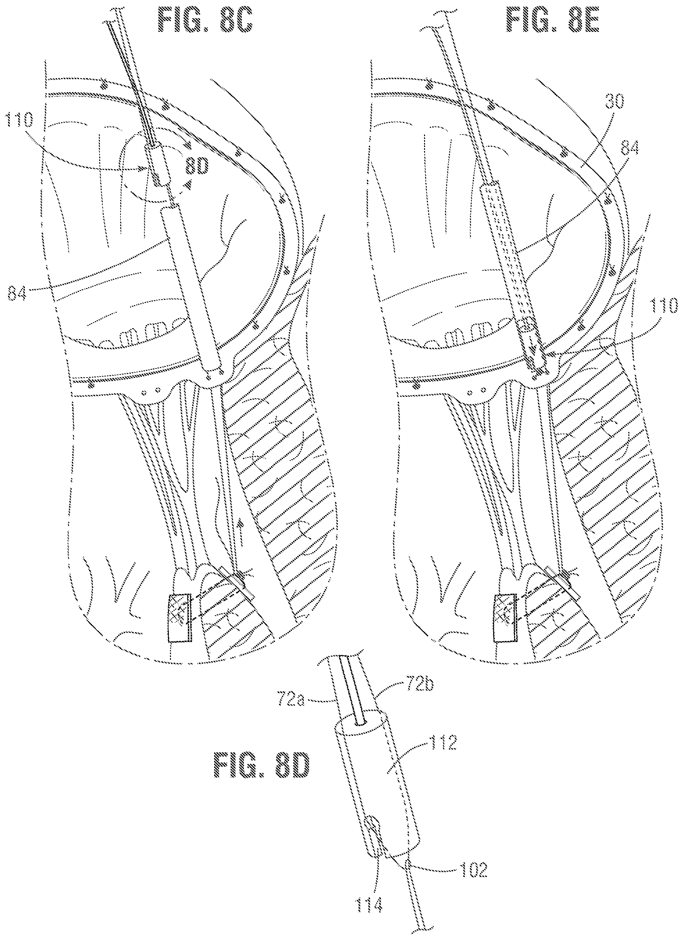

Figure 8B shows a loosely-formed single knot 102 between the two free ends 72a, 72b of the tensioning suture. This operation is accomplished outside the body for convenience. Figures 8C and 8D show engagement of a knot pusher 110 with the loose single knot 102 the knot pusher 110 has an enlarged head 112 with a distal end bifurcated by a lateral slit 114 in which the loose single knot 102 may be captured with the free ends 72a, 72b extending proximally therefrom. The knot pusher 110 further includes a flexible shaft 116 with column strength so that the knot 102 may be pushed through the respective tubular arm 84. Slight traction is applied to the slack suture 72a as the knot pusher 110 is advanced into and through the tube 84. Figure 8E shows a final step where the knot 102 has been pushed through the arm 84 into contact with the proximal side of the annuloplasty ring 30 at the boss 60. This operation is repeated to install between about 5-15 knots to ensure that they will not come untied. The first two sutures could be placed in the same direction as a slip knot if desired, therefore allowing for some further tension adjustment, or in alternate directions to lock them. Once tied, both knots 76, 102 below and above the mitral annulus MA will carry the load of the PM and thus offer strength and durability in the long term. Knots are routinely tied through trocars in much the same way in laparoscopic surgery procedures in general surgery, orthopedics, obstetrics-gynecology, *etc.*

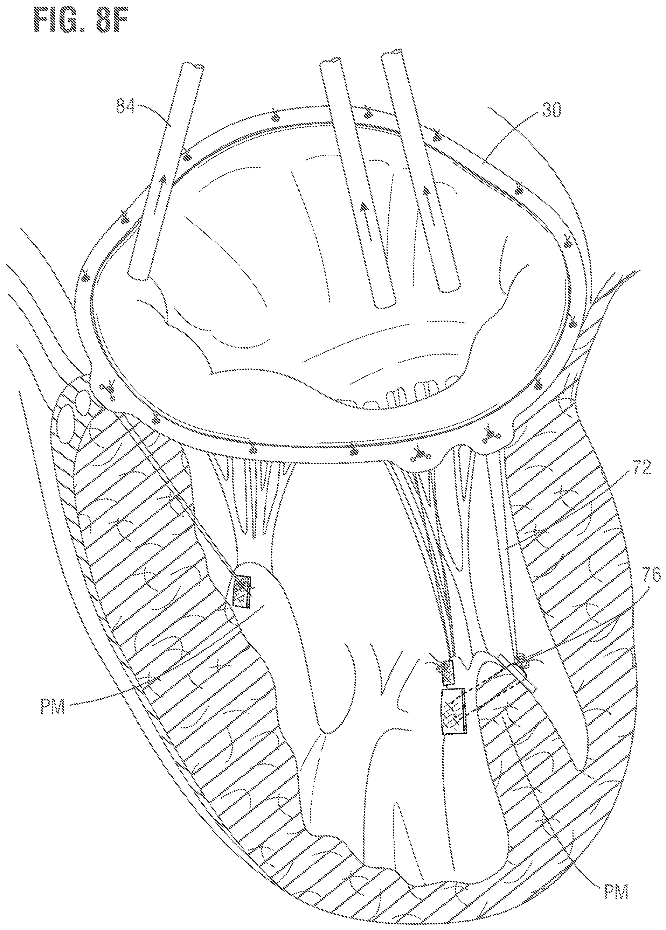

Once each of the tethering sutures 72 has been secured to the proximal side of the annuloplasty ring 30, the sutures are severed close to the ring with a guillotine-type cutting mechanism or other cutting device. The tether adjustment system arms 84 are then removed from the body, as seen in Figure 8F. As the tether adjustment shaft 94 seen in Figure 7 is removed from the atrial wall, the purse-string suture 96 is tightened to close off the access hole. The remaining access incisions leading to the atrial wall are also closed.

Figure 8F shows the annuloplasty ring 30 securely anchored around the mitral annulus MA with one or more tethering sutures 72 in tension with subvalvular structures such as the papillary muscles PM. As explained, annuloplasty ring 30 may cause a remodeling or constriction of the mitral annulus MA, or may simply be used as a fixed anchor for the tethering sutures 72 if the mitral annulus is otherwise in good shape.

It should be understood that tethering the annuloplasty ring 30 to the papillary muscles PM is only one technique that can be used. In addition to the configuration shown, various other means of attachment to the PMs or heart wall are possible, such as helical anchors with sutures extending from them, for example, or anchors mounted to the exterior of the heart with sutures extending through the myocardium and toward the annulus. Simple pledgets on the exterior surface of the heart could also be used, or knots could be deployed on the outside of the heart with their sutures extending back into the heart toward the annulus. Other types of anchors not explicitly mentioned here should also be included. For instance, WO2020197854A1 to Passman, et al. discloses a number of ventricular anchors within or to the exterior of the heart muscle which could be utilized.

Also, a modular approach to the delivery system could be used wherein the tension adjustment system tubes 84 and tension adjusters 98 are separate, rather than merging into a single tube and then splitting out again near the system handle. With separate systems, there would be more flexibility in how many PM relocation sutures were used. The system could, for example, come with enough for 4 sets of tubes with tension adjusters and the surgeon could decide how many they needed to use depending on the individual patient's anatomy. With 4 sets of tubes, for example, a single barrel-shaped component could be included that contained 4 hemostatic valves such that the tubes to be used could be passed through the valves, and then the barrel-shaped component could be used for hemostatic seal at the atrial wall with a purse-string, rather than a common tube as shown in the attached figures.

Likewise, various connections between the annuloplasty ring 30 and the PM suture tension tubes 84 are possible. Araised male feature could be part of each boss that mates with the end of the tube 84, potentially with a snap fit for security. A sleeve within each tube 84 could then "eject" the ring 30 at its connection when appropriate by, for example, pushing a control on the delivery system handle. Other connections such as threaded connections are possible. Since there is tension in the PM relocation sutures and, hence, compression between the tubes and ring core, a connection with a mechanical lock may not be necessary, however.

While the foregoing is a complete description of the preferred examples, various alternatives, modifications, and equivalents may be used. Moreover, it will be obvious that certain other modifications may be practiced.

## Claims

1. An annuloplasty ring and tether adjustment system, comprising:
an annuloplasty ring (30) including an inner core (40) covered by an outer suture-permeable interface (42), the inner core (40) defining a closed peripheral shape surrounding an orifice and the interface (42) closely surrounding the inner core (40) and having a matching shape, the inner core (40) defining at least one outwardly-projecting boss (60, 62, 64, 66) configured to provide a suture anchor;
a plurality of anchoring sutures (70) adapted to pass through the interface (42) and secure the annuloplasty ring (30) to a native heart valve annulus;
at least one tethering suture (72) configured to loop through subvalvular structure in a chamber below the native heart valve annulus and tie off thereto, the tethering suture (72) having a length such that a pair of free ends extends from the subvalvular structure through the outwardly-projecting boss (60, 62, 64, 66) on the inner core (40) and out of the body;
a tether adjusting mechanism (90) including at least one tubular arm (84) having a length sufficient to extend from the native heart valve annulus through an access incision in the heart and out of the body, the pair of free ends (72a, 72b) of the at least one tethering suture (72) passing through the at least one tubular arm (84), the pair of free ends having a first end (72a) and a second end (72b); and
a tensioner (98) that receives the second end (72b), the at least one tubular arm (84) having sufficient column strength such that tension on the second end (72b) caused by the tensioner (98) decreases the distance between the subvalvular structure and the annuloplasty ring (30), at which point the first and second ends (72a, 72b) are configured to tie off to the outwardly-projecting boss (60, 62, 64, 66).

2. The system of claim 1, wherein there are at least two outwardly-projecting bosses (60, 64) spaced apart around the inner core (40b).

3. The system of claim 1, wherein there are three outwardly-projecting bosses (60, 62, 64) spaced apart around the inner core (40a; 40d), two of which are more closely spaced to each other than from a third.

4. The system of any previous claim, wherein the interface (42) includes a silicone inner portion surrounded by a fabric cover.

5. The system of any previous claim, wherein the annuloplasty ring (30) is adapted to be implanted at the mitral annulus, and the inner core peripheral shape is a rounded D-shape with an anterior segment (50) on a substantially straight side opposite a more curved posterior segment (52), and wherein the at least one outwardly-projecting boss (60, 62, 64, 66) is located in the posterior segment (52).

6. The system of claim 5, wherein the inner core (40) defines a major axis (X) across a long dimension perpendicular to a minor axis (Y), wherein the anterior segment (50) and the posterior segment (52) are on opposite sides of the major axis (X), and wherein there are at least two outwardly-projecting bosses (60, 62, 64, 66) on opposite sides of the minor axis (Y) and located in the posterior segment (52).

7. The system of claim 6, wherein there are only two outwardly-projecting bosses (60, 64), one on each side of the minor axis (Y) and two tethering sutures (72).

8. The system of any previous claim, wherein the tether adjusting mechanism (90) includes a tubular arm (84) for each tethering suture (72), and further including a common access sheath through which the tubular arms (84) extend.

9. The system of claim 8, further including a knot pusher (110) for advancing knots (102) in each tethering suture (72) through a respective tubular arm (84) to a respective outwardly-projecting boss (60, 62, 64, 66) on the annuloplasty ring (30).

10. The system of any previous claim, wherein the at least one outwardly-projecting boss (60, 62, 64) comprises a rounded rectangular projection through which a pair of identical apertures (68) extend.

11. The system of any of claims 1 to 9, wherein the at least one outwardly-projecting boss (66) comprises a T-shaped projection having rounded ends.

12. A method of simulating implantation of an annuloplasty ring in a simulated patient at a native heart valve annulus and adjusting subvalvular structure below the native heart valve annulus, comprising:
stopping a simulated patient's heart and providing an access pathway to a native heart valve annulus though a body to the heart, including an access incision in the model of the heart leading to a heart valve annulus;
looping a plurality of anchoring sutures (70) through the heart valve annulus at spaced locations and retracting free ends of the anchoring sutures (70) out of the body;
looping at least one tethering suture (72) through a subvalvular structure below the heart valve annulus and retracting free ends of the tethering suture (72) out of the body;
passing one end of each of the pairs of free ends (72a, 72b) of the anchoring sutures (72) through a peripheral suture-permeable interface (42) of an annuloplasty ring (30), the annuloplasty ring (30) including an inner core (40) defining a closed peripheral shape surrounding an orifice and the interface (42) closely surrounding the inner core (40) and having a matching shape, the inner core (40) defining at least one outwardly-projecting boss (60, 62, 64, 66) configured to provide a suture anchor;
passing both free ends (72a, 72b) of the at least one tethering suture (72) upward through the outwardly-projecting boss (60, 62, 64, 66) and retracting the free ends (72a, 72b) of the at least one tethering suture (72) out of the body;
advancing the annuloplasty ring (30) to the heart valve annulus along the anchoring sutures (72);
tying off the anchoring sutures (72) on a proximal side of the annuloplasty ring (30);
sealing the access incision around the free ends (72a, 72b) of the at least one tethering suture (72);
restarting the patient's heart;
externally visualizing blood flow through the heart valve annulus;
adjusting the tension on one of the free ends (72a, 72b) of the at least one tethering suture (72);
securing the free ends (72a, 72b) of the at least one tethering suture (72) on the proximal side of the annuloplasty ring at the respective outwardly-projecting boss (60, 62, 64, 66), and severing the free ends (72a, 72b) of the at least one tethering suture (72); and
withdrawing the at least one tethering suture (72) from the body and closing up the access pathway.

13. The method of claim 12, wherein after the step of advancing the annuloplasty ring (30) to the heart valve annulus, the method includes advancing a tubular arm (84) down each pair of free ends (72a, 72b) of tethering sutures (72) from outside the body until the tubular arm (84) contacts the annuloplasty ring (30), and wherein the step of adjusting the tension on one of the free ends (72a, 72b) of the at least one tethering suture (72) places the tubular arm (84) in compression; in particular further including sequentially advancing knots in each tethering suture through an associated tubular arm to an associated outwardly-projecting boss on the annuloplasty ring using a knot pusher.

14. The method of claim 13, wherein the tubular arms pass through a common access sheath, and the access incision is sealed around the common access sheath; or wherein each pair of free ends has a first end and a second end, and further including a tensioner that receives the second end, the at least one tubular arm having sufficient column strength such that tension on the second end caused by the tensioner decreases the distance between the subvalvular structure and the annuloplasty ring, at which point the first and second end are configured to tie off to the outwardly-projecting boss.

15. The method of any of claims 12 to 13, wherein the at least one outwardly-projecting boss (60, 62, 64) comprises a rounded rectangular projection through which a pair of identical apertures (68) extend; and/or wherein the at least one outwardly-projecting boss (66) comprises a T-shaped projection having rounded ends.

## Patentansprüche

1. System zum Einstellen eines Anuloplastie-Rings und einer Halteleine, umfassend:
einen Annuloplastie-Ring (30) mit einem inneren Kern (40), der von einer äußeren, für Nahtfäden durchlässigen Grenzfläche (42) bedeckt ist, wobei der innere Kern (40) eine geschlossene umlaufende Form definiert, die eine Öffnung umgibt, und die Grenzfläche (42) den inneren Kern (40) eng umgibt und eine passende Form aufweist, wobei der innere Kern (40) wenigstens einen nach außen hervorstehenden Ansatz (60, 62, 64, 66) definiert, der dazu konfiguriert ist, einen Fadenanker bereitzustellen;
mehrere Verankerungsfäden (70), die dazu eingerichtet sind, durch die Grenzfläche (42) zu verlaufen und den Anuloplastie-Ring (30) an einem nativen Herzklappenanulus zu befestigen;
wenigstens einen Haltefaden (72), der dazu konfiguriert ist, sich durch die subvalvuläre Struktur in einer Kammer unterhalb des nativen Herzklappenanulus zu schlingen und daran festzumachen, wobei der Haltefaden (72) eine solche Länge aufweist, dass sich ein Paar freier Enden von der subvalvulären Struktur durch den nach außen hervorstehenden Ansatz (60, 62, 64, 66) am inneren Kern (40) und aus dem Körper heraus erstreckt;
eine Halteleine-Einstelleinrichtung (90), die wenigstens einen röhrenförmigen Arm (84) mit einer Länge aufweist, die ausreicht, um sich vom nativen Herzklappenanulus durch einen Zugangsschnitt im Herzen und aus dem Körper heraus zu erstrecken, wobei das Paar freier Enden (72a, 72b) des wenigstens einen Haltefadens (72) durch den wenigstens einen röhrenförmigen Arm (84) verläuft, wobei das Paar freier Enden ein erstes Ende (72a) und ein zweites Ende (72b) aufweist; und
eine Spanneinrichtung (98), die das zweite Ende (72b) aufnimmt, wobei der wenigstens eine röhrenförmige Arm (84) eine ausreichende Knickfestigkeit aufweist, so dass die durch die Spanneinrichtung (98) verursachte Spannung am zweiten Ende (72b) den Abstand zwischen der subvalvulären Struktur und dem Anuloplastie-Ring (30) verringert, wobei an diesem Punkt das erste und das zweite Ende (72a, 72b) dazu konfiguriert sind, an dem nach außen hervorstehenden Ansatz (60, 62, 64, 66) festzumachen.

2. System nach Anspruch 1, wobei wenigstens zwei nach außen hervorstehende Ansätze (60, 64) im Abstand um den inneren Kern (40b) herum angeordnet sind.

3. System nach Anspruch 1, wobei drei nach außen hervorstehende Ansätze (60, 62, 64) im Abstand um den inneren Kern (40a; 40d) herum angeordnet sind, von denen zwei einen geringeren Abstand zueinander als zu einem dritten aufweisen.

4. System nach einem der vorangehenden Ansprüche, wobei die Grenzfläche (42) einen inneren Abschnitt aus Silikon aufweist, der von einer Stoff-Abdeckung umgeben ist.

5. System nach einem der vorangehenden Ansprüche, wobei der Anuloplastie-Ring (30) dazu eingerichtet ist, am Mitral-Anulus implantiert zu werden, und die umlaufende Form des inneren Kerns eine abgerundete D-Form ist, mit einem anterioren Segment (50) auf einer im Wesentlichen geraden Seite gegenüber einem stärker gekrümmten posterioren Segment (52), und wobei der wenigstens eine nach außen hervorstehende Ansatz (60, 62, 64, 66) in dem posterioren Segment (52) angeordnet ist.

6. System nach Anspruch 5, wobei der innere Kern (40) eine Hauptachse (X) über eine lange Abmessung senkrecht zu einer Nebenachse (Y) definiert, wobei sich das anteriore Segment (50) und das posteriore Segment (52) auf gegenüberliegenden Seiten der Hauptachse (X) befinden, und wobei es wenigstens zwei nach außen hervorstehende Ansätze (60, 62, 64, 66) auf gegenüberliegenden Seiten der Nebenachse (Y) gibt, die im posterioren Segment (52) angeordnet sind.

7. System nach Anspruch 6, wobei nur zwei nach außen hervorstehende Ansätze (60, 64) vorhanden sind, einer auf jeder Seite der Nebenachse (Y), und zwei Haltefäden (72).

8. System nach einem der vorangehenden Ansprüche, wobei die Halteleine-Einstelleinrichtung (90) für jeden Haltefaden (72) einen röhrenförmigen Arm (84) aufweist und ferner eine gemeinsame Zugangshülle aufweist, durch die sich die röhrenförmigen Arme (84) erstrecken.

9. System nach Anspruch 8, das ferner einen Knotenschieber (110) zum Vorschieben von Knoten (102) in jedem Haltefaden (72) durch einen entsprechenden röhrenförmigen Arm (84) zu einem entsprechenden nach außen hervorstehenden Ansatz (60, 62, 64, 66) auf dem Anuloplastie-Ring (30) umfasst.

10. System nach einem der vorangehenden Ansprüche, wobei der wenigstens eine nach außen hervorstehende Ansatz (60, 62, 64) einen abgerundeten rechteckigen Vorsprung umfasst, durch den sich ein Paar identischer Öffnungen (68) erstreckt.

11. System nach einem der Ansprüche 1 bis 9, wobei der wenigstens eine nach außen hervorstehende Ansatz (66) einen T-förmigen Vorsprung mit abgerundeten Enden aufweist.

12. Verfahren zur Simulation der Implantation eines Annuloplastie-Rings in einem simulierten Patienten an einem nativen Herzklappenanulus und zum Einstellen der subvalvulären Struktur unterhalb des nativen Herzklappenanulus, umfassend:
Anhalten eines simulierten Patientenherzens und Bereitstellen eines Zugangsweges zu einem nativen Klappenanulus durch einen Körper zu dem Herzen, einschließlich eines Zugangsschnittes in dem Modell des Herzens, der zu einem Herzklappenanulus führt;
Schlingen mehrerer Verankerungsfäden (70) durch den Herzklappenanulus an beabstandeten Stellen und Zurückziehen der freien Enden der Verankerungsfäden (70) aus dem Körper;
Schlingen von wenigstens einem Haltefaden (72) durch eine subvalvuläre Struktur unterhalb des Herzklappenanulus und Zurückziehen der freien Enden des Haltefadens (72) aus dem Körper;
Durchführen eines Endes jedes der Paare von freien Enden (72a, 72b) der Verankerungsfäden (72) durch eine periphere, für Nahtfäden durchlässige Grenzfläche (42) eines Annuloplastie-Rings (30), wobei der Annuloplastie-Ring (30) einen inneren Kern (40) aufweist, der eine geschlossene umlaufende Form definiert, die eine Öffnung umgibt, und wobei die Grenzfläche (42) den inneren Kern (40) eng umgibt und eine passende Form aufweist, wobei der innere Kern (40) wenigstens einen nach außen hervorstehenden Ansatz (60, 62, 64, 66) aufweist, der dazu konfiguriert ist, einen Fadenanker bereitzustellen;
Durchführen der beiden freien Enden (72a, 72b) des wenigstens einen Haltefadens (72) nach oben durch den nach außen hervorstehenden Ansatz (60, 62, 64, 66) und Zurückziehen der freien Enden (72a, 72b) des wenigstens einen Haltefadens (72) aus dem Körper;
Vorschieben des Anuloplastie-Rings (30) zum Herzklappenanulus entlang der Verankerungsfäden (72);
Festmachen der Verankerungsfäden (72) an einer proximalen Seite des Anuloplastie-Rings (30);
Abdichten des Zugangsschnittes um die freien Enden (72a, 72b) des wenigstens einen Haltefadens (72);
Reaktivierung des Herzens des Patienten;
externe Visualisierung des Blutflusses durch den Herzklappenanulus;
Einstellen der Spannung an einem der freien Enden (72a, 72b) des wenigstens einen Haltefadens (72);
Befestigen der freien Enden (72a, 72b) des wenigstens einen Haltefadens (72) an der proximalen Seite des Anuloplastie-Rings an dem entsprechenden nach außen hervorstehenden Ansatz (60, 62, 64, 66), und Abtrennen der freien Enden (72a, 72b) des wenigstens einen Haltefadens (72); und
Zurückziehen des wenigstens einen Haltefadens (72) aus dem Körper und Verschließen des Zugangsweges.

13. Verfahren nach Anspruch 12, wobei nach dem Schritt des Vorschiebens des Annuloplastie-Rings (30) zum Herzklappenanulus das Verfahren das Vorschieben eines röhrenförmigen Arms (84) entlang jedes Paares von freien Enden (72a, 72b) der Haltefäden (72) von außerhalb des Körpers, bis der röhrenförmige Arm (84) den Anuloplastie-Ring (30) berührt, umfasst und wobei der Schritt des Einstellens der Spannung an einem der freien Enden (72a, 72b) des wenigstens einen Haltefadens (72) den röhrenförmigen Arm (84) in Kompression versetzt; insbesondere einschließlich des sequentiellen Vorschiebens von Knoten in jedem Haltefaden durch einen zugehörigen röhrenförmigen Arm zu einem zugehörigen, nach außen hervorstehenden Ansatz auf dem Annuloplastie-Ring unter Verwendung eines Knotenschiebers.

14. Verfahren nach Anspruch 13, wobei die röhrenförmigen Arme durch eine gemeinsame Zugangshülle verlaufen und der Zugangsschnitt um die gemeinsame Zugangshülle herum abgedichtet ist; oder wobei jedes Paar freier Enden ein erstes Ende und ein zweites Ende aufweist und ferner eine Spanneinrichtung enthält, die das zweite Ende aufnimmt, wobei der wenigstens eine röhrenförmige Arm eine ausreichende Knickfestigkeit aufweist, so dass die durch die Spanneinrichtung verursachte Spannung am zweiten Ende den Abstand zwischen der subvalvulären Struktur und dem Anuloplastie-Ring verringert, wobei an diesem Punkt das erste und das zweite Ende dazu konfiguriert sind, an dem nach außen hervorstehenden Ansatz festzumachen.

15. Verfahren nach einem der Ansprüche 12 bis 13, wobei der wenigstens eine nach außen hervorstehende Ansatz (60, 62, 64) einen abgerundeten rechteckigen Vorsprung umfasst, durch den sich ein Paar identischer Öffnungen (68) erstreckt; und/oder wobei der wenigstens eine nach außen hervorstehende Ansatz (66) einen T-förmigen Vorsprung mit abgerundeten Enden umfasst.

## Revendications

1. Système d'anneau d'annuloplastie et d'ajustement d'attache, comprenant :
un anneau d'annuloplastie (30) comprenant un noyau interne (40) recouvert d'une interface externe (42) perméable à la suture, le noyau interne (40) définissant une forme périphérique fermée entourant un orifice et l'interface (42) entourant étroitement le noyau interne (40) et présentant une forme appariée, le noyau interne (40) définissant au moins un bossage (60, 62, 64, 66) faisant saillie vers l'extérieur conçu pour fournir un ancrage de suture ;
une pluralité de sutures d'ancrage (70) conçues pour passer à travers l'interface (42) et fixer l'anneau d'annuloplastie (30) à un anneau de valvule cardiaque native ;
au moins une suture d'attache (72) conçue pour boucler à travers la structure sous-valvulaire dans une chambre au-dessous de l'anneau de valvule cardiaque native et se ligaturer à celle-ci, la suture d'attache (72) présentant une longueur telle qu'une paire d'extrémités libres s'étend à partir de la structure sous-valvulaire à travers le bossage (60, 62, 64, 66) faisant saillie vers l'extérieur sur le noyau interne (40) et hors du corps ;
un mécanisme d'ajustement (90) d'attache comprenant au moins un bras tubulaire (84) présentant une longueur suffisante pour s'étendre à partir de l'anneau de valvule cardiaque native à travers une incision d'accès dans le cœur et hors du corps, la paire d'extrémités libres (72a, 72b) de ladite au moins une suture d'attache (72) passant à travers ledit au moins un bras tubulaire (84), la paire d'extrémités libres présentant une première extrémité (72a) et une seconde extrémité (72b) ; et
un tendeur (98) qui reçoit la seconde extrémité (72b), ledit au moins un bras tubulaire (84) présentant une résistance en colonne suffisante de telle sorte qu'une tension sur la seconde extrémité (72b) provoquée par le tendeur (98) diminue la distance entre la structure sous-valvulaire et l'anneau d'annuloplastie (30), à la suite de quoi les première et seconde extrémités (72a, 72b) sont conçues pour se ligaturer au bossage (60, 62, 64, 66) faisant saillie vers l'extérieur.

2. Système selon la revendication 1, dans lequel il existe au moins deux bossages (60, 64) faisant saillie vers l'extérieur espacés autour du noyau interne (40b).

3. Système selon la revendication 1, dans lequel il existe trois bossages (60, 62, 64) faisant saillie vers l'extérieur espacés autour du noyau interne (40a ; 40d), dont deux sont plus rapprochés l'un de l'autre que d'un troisième.

4. Système selon l'une quelconque revendication précédente, dans lequel l'interface (42) comprend une partie interne en silicone entourée par un revêtement en tissu.

5. Système selon l'une quelconque revendication précédente, dans lequel l'anneau d'annuloplastie (30) est conçu pour être implanté au niveau de l'anneau mitral et la forme périphérique de noyau interne est une forme arrondie en D présentant un segment antérieur (50) sur un côté sensiblement droit opposé à un segment postérieur (52) plus incurvé et dans lequel ledit au moins un bossage (60, 62, 64, 66) faisant saillie vers l'extérieur est situé dans le segment postérieur (52).

6. Système selon la revendication 5, dans lequel le noyau interne (40) définit un grand axe (X) sur une longue dimension perpendiculaire à un petit axe (Y), dans lequel le segment antérieur (50) et le segment postérieur (52) sont sur des côtés opposés du grand axe (X) et dans lequel il existe au moins deux bossages (60, 62, 64, 66) faisant saillie vers l'extérieur sur des côtés opposés du petit axe (Y) et situés dans le segment postérieur (52).

7. Système selon la revendication 6, dans lequel il n'existe que deux bossages (60, 64) faisant saillie vers l'extérieur, un de chaque côté du petit axe (Y) et deux sutures d'attache (72).

8. Système selon l'une quelconque revendication précédente, dans lequel le mécanisme d'ajustement (90) d'attache comprend un bras tubulaire (84) pour chaque suture d'attache (72) et comprenant en outre une gaine d'accès commune à travers laquelle s'étendent les bras tubulaires (84).

9. Système selon la revendication 8, comprenant en outre un poussoir de nœud (110) destiné à faire avancer des nœuds (102) dans chaque suture d'attache (72) à travers un bras tubulaire (84) respectif vers un bossage (60, 62, 64, 66) faisant saillie vers l'extérieur respectif sur l'anneau d'annuloplastie (30).

10. Système selon l'une quelconque revendication précédente, dans lequel ledit au moins un bossage (60, 62, 64) faisant saillie vers l'extérieur comprend une saillie rectangulaire arrondie à travers laquelle s'étend une paire d'ouvertures (68) identiques.

11. Système selon l'une quelconque des revendications 1 à 9, dans lequel ledit au moins un bossage (66) faisant saillie vers l'extérieur comprend une saillie en forme de T présentant des extrémités arrondies.

12. Procédé de simulation de l'implantation d'un anneau d'annuloplastie chez un patient simulé au niveau d'un anneau de valvule cardiaque native et d'ajustement de la structure sous-valvulaire au-dessous de l'anneau de valvule cardiaque native, comprenant :
l'arrêt du cœur d'un patient simulé et la réalisation d'une voie d'accès à un anneau de valvule cardiaque native à travers un corps vers le cœur, comprenant une incision d'accès dans le modèle du cœur conduisant à un anneau de valvule cardiaque ;
le bouclage d'une pluralité de sutures d'ancrage (70) à travers l'anneau de valvule cardiaque à des emplacements espacés et la rétraction des extrémités libres des sutures d'ancrage (70) hors du corps ;
le bouclage d'au moins une suture d'attache (72) à travers une structure sous-valvulaire au-dessous de l'anneau de valvule cardiaque et la rétraction des extrémités libres de la suture d'attache (72) hors du corps ;
le passage d'une extrémité de chacune des paires d'extrémités libres (72a, 72b) des sutures d'ancrage (72) à travers une interface périphérique (42) perméable à la suture d'un anneau d'annuloplastie (30), l'anneau d'annuloplastie (30) comprenant un noyau interne (40) définissant une forme périphérique fermée entourant un orifice et l'interface (42) entourant étroitement le noyau interne (40) et présentant une forme appariée, le noyau interne (40) définissant au moins un bossage (60, 62, 64, 66) faisant saillie vers l'extérieur conçu pour fournir un ancrage de suture ;
le passage des deux extrémités libres (72a, 72b) de ladite au moins une suture d'attache (72) vers le haut à travers le bossage (60, 62, 64, 66) faisant saillie vers l'extérieur et la rétraction des extrémités libres (72a, 72b) de ladite au moins une suture d'attache (72) hors du corps ;
l'avancement de l'anneau d'annuloplastie (30) vers l'anneau de valvule cardiaque le long des sutures d'ancrage (72) ;
la ligature des sutures d'ancrage (72) sur un côté proximal de l'anneau d'annuloplastie (30) ;
la fermeture hermétique de l'incision d'accès autour des extrémités libres (72a, 72b) de ladite au moins une suture d'attache (72) ;
le redémarrage du cœur du patient ;
la visualisation externe du flux sanguin à travers l'anneau de valvule cardiaque ;
l'ajustement de la tension sur l'une des extrémités libres (72a, 72b) de ladite au moins une suture d'attache (72) ;
la fixation des extrémités libres (72a, 72b) de ladite au moins une suture d'attache (72) sur le côté proximal de l'anneau d'annuloplastie au niveau du bossage (60, 62, 64, 66) faisant saillie vers l'extérieur respectif et le sectionnement des extrémités libres (72a, 72b) de ladite au moins une suture d'attache (72) ; et
le retrait de ladite au moins une suture d'attache (72) du corps et la fermeture de la voie d'accès.

13. Procédé selon la revendication 12, dans lequel, après l'étape d'avancement de l'anneau d'annuloplastie (30) vers l'anneau de valvule cardiaque, le procédé comprend l'avancement d'un bras tubulaire (84) vers le bas de chaque paire d'extrémités libres (72a, 72b) de sutures d'attache (72) depuis l'extérieur du corps jusqu'à ce que le bras tubulaire (84) entre en contact avec l'anneau d'annuloplastie (30) et dans lequel l'étape d'ajustement de la tension sur l'une des extrémités libres (72a, 72b) de ladite au moins une suture d'attache (72) place le bras tubulaire (84) en compression ; en particulier comprenant en outre l'avancement séquentiel de nœuds dans chaque suture d'attache à travers un bras tubulaire associé vers un bossage faisant saillie vers l'extérieur associé sur l'anneau d'annuloplastie à l'aide d'un poussoir de nœud.

14. Procédé selon la revendication 13, dans lequel les bras tubulaires passent à travers une gaine d'accès commune et l'incision d'accès est fermée hermétiquement autour de la gaine d'accès commune ; ou dans lequel chaque paire d'extrémités libres présente une première extrémité et une seconde extrémité et comprenant en outre un tendeur qui reçoit la seconde extrémité, ledit au moins un bras tubulaire présentant une résistance en colonne suffisante de telle sorte qu'une tension sur la seconde extrémité provoquée par le tendeur diminue la distance entre la structure sous-valvulaire et l'anneau d'annuloplastie, à la suite de quoi les première et seconde extrémités sont conçues pour se ligaturer au bossage faisant saillie vers l'extérieur.

15. Procédé selon l'une quelconque des revendications 12 à 13, dans lequel ledit au moins un bossage (60, 62, 64) faisant saillie vers l'extérieur comprend une saillie rectangulaire arrondie à travers laquelle s'étend une paire d'ouvertures (68) identiques ; et/ou dans lequel ledit au moins un bossage (66) faisant saillie vers l'extérieur comprend une saillie en forme de T présentant des extrémités arrondies.
